# EUROPÄISCHE PATENTSCHRIFT

(11) **EP 1 515 922 B1**
(45) Veröffentlichungstag und Bekanntmachung des Hinweises auf die Patenterteilung: **24.05.2006**
(21) Anmeldenummer: 03735675.5
(22) Anmeldetag: 24.06.2003
(51) Int. Cl.: C03C 3/097, C03C 4/00, A61K 6/06

(54) **OPALESZIERENDE GLASKERAMIK**
OPALESCENT GLASS-CERAMIC PRODUCT
VITROCERAMIQUE OPALESCENTE

(30) Priorität: 25.06.2002 DE 10228381
(43) Veröffentlichungstag der Anmeldung: 23.03.2005
(73) Patentinhaber: DeguDent GmbH, 63457 Hanau (DE)
(72) Erfinder: KRUMBHOLZ, Klaus, 63225 Langen (DE)
(74) Vertreter: Stoffregen, Hans-Herbert
(86) Internationale Anmeldenummer: PCT/EP2003/006644
(87) Internationale Veröffentlichungsnummer: WO 2004/000743

(56) Entgegenhaltungen:
- EP-A- 0 622 342
- EP-A- 0 885 855
- US-A- 6 022 819
- US-B1- 6 280 863

## Beschreibung

Die Erfindung bezieht sich auf eine opaleszierende Glaskeramik, insbesondere eine opaleszierende Glaskeramik als Dentalmaterial oder als Zusatz zu oder Bestandteil von Dentalmaterial, enthaltend zumindest die Komponenten SiO₂, Al₂O₃, P₂O₅, Na₂O, K₂O, CaO sowie Me(IV)O₂. Ferner bezieht sich die Erfindung auf ein Verfahren zur Herstellung einer opaleszierenden Glaskeramik sowie auf die Verwendung einer solchen.

Opaleszierende Gläser sind beispielsweise aus der EP 0 622 342 B1 bekannt. In der EP 0 622 342 B1 wird ein opaleszierendes Glas auf der Basis von SiO₂- B₂O₃ - Al₂O₃ - K₂O - Na₂O - CaO - BaO - SrO - TiO₂ - ZrO₂ - P₂O₅ - CeO₂ offenbart. Die dort angegebenen linearen Wärmeausdehnungskoeffizienten (WAK) sind entweder viel zu hoch (Beispiele 5, 15, 26, 27 mit WAK ≥ 15, 1) oder viel zu niedrig (WAK ≤ 10,8), um für die bevorzugte Verwendung des Materials als Verblendkeramik für metallische Zahnrestaurationen in reiner Form einsetzbar zu sein. Für die Verblendung gängiger Legierungen ist ein Verschneiden mit weiteren Gläsern notwendig.

Außerdem haben Versuche ergeben, dass die aus EP 0 622 342 B1 bekannten Glaskeramiken zu trüb sind, so dass eine gute Ästhetik nicht gewährleistet ist. Insgesamt zeigen die aus EP 0 622 342 B1 bekannten opaleszierenden Glaskeramiken zu schwache und nur geringe brennstabile Opaleszenz, eine zu hohe Opazität und weisen keine Fluoreszenz auf.

Aus der US 6,022,819 ist ein Porzellanwerkstoff bestimmt für den Dentalbereich bekannt. Dieser weist als Bestandteile TiO₂ bzw. ZrO₂ auf. Der SiO₂-Anteil beträgt vorzugsweise 50 bis 85 Gew.-%. Ebenfalls TiO₂ - und ZrO₂-haltige opaleszierende Dental-Glaskeramiken sind der EP-A-0 885 855 und der US-B-6 280 863 zu entnehmen.

Der vorliegenden Erfindung liegt das Problem zu Grunde, eine opaleszierende Glaskeramik, ein Verfahren zu ihrer Herstellung sowie ihre Verwendung zur Verfügung zu stellen, das eine verbesserte Opaleszenz bei gleichzeitig verbesserter Transparenz und eine auf andere Materialien abgestimmte Brenntemperatur bzw. Wärmeausdehnungskoeffizienten sowie Fluoreszenz aufweist.

Das Problem wird erfindungsgemäß durch eine opaleszierende Glaskeramik der eingangs genannten Art im Wesentlichen dadurch gelöst, dass die opaleszierende Glaskeramik ZrO₂ und TiO₂ frei ist, dass die Glaskeramik einen Me(II)O-Gehalt von weniger als 4 Gew.-% aufweist und dass der Me(IV) O₂-Gehalt 0,5 bis in etwa 3 Gew.-% beträgt. In bevorzugter Weise ist vorgesehen, dass sich der Me(IV) O₂ - Gehalt aus 0 - 1 Gew.-% CeO₂ und 0 - 2,5 Gew.-% SnO₂ zusammensetzt.

Insbesondere beträgt der Me (II)O-Gehalt 2 - 3,5 Gew.-%, vorzugsweise 2,5 - 3 Gew.-%.

Eine bevorzugte Zusammensetzung enthält die folgenden Komponenten:

| Komponente | Gew.-% |
|---|---|
| SiO₂ | 55 - 62 |
| Al₂O₃ | 13-17 |
| B₂O₃ | 0 - 2 |
| P₂O₅ | 1,5 - 3 |
| Li₂O | 0 - 2 |
| Na₂O | 7-12 |
| K₂O | 8 -12 |
| MgO | 0-2 |
| CaO | 1-4 |
| BaO | 0-2 |
| Tb₂O₃ | 0 - 3 |
| Me(IV)O₂ | 0,5 - 3 |

wobei die angegebene Menge Me(IV)O₂ aus 0 - 1 Gew.-% CeO₂ und 0 - 2,5 Gew.-% SnO₂ gebildet ist.

Insbesondere zeichnet sich die Zusammensetzung der Glaskeramik durch folgende Komponenten aus:

| Komponente | Gew.-% |
|---|---|
| SiO₂ | 58 - 60 |
| Al₂O₃ | 14 - 15 |
| P₂O₅ | 2,3 - 2,6 |
| Na₂O | 9,5 - 10,5 |
| K₂O | 9 - 10 |
| CaO | 2,8 - 3,0 |
| SnO₂ | 1,3 - 1,6 |
| CeO₂ | 0,3 - 0,4 |
| Tb₂O₃ | 0 - 2,0 |

Erfindungsgemäß wird eine verbesserte Opaleszenz durch Entmischungen der Gläser auf Grund von P₂O₅ - und SnO₂ - Gehalten erreicht. Der Wärmeausdehnungskoeffizient (WAK) der erfindungsgemäßen Keramik liegt im Bereich von 9,0 - 13,5 x 10⁻⁶/K und kann über den K₂O-Gehalt gesteuert werden.

Durch zusätzliches Einschnielzen von CeO₂ und/oder Tb₂O₃ kann erreicht werden, dass die Glaskeramik fluoresziert, eine für Dentalkeramik wünschenswerte Eigenschaft. Durch eine Kombination beider Oxide wird eine starke, neutrale Fluoreszenz erreicht.

Des Weiteren kann die anwendungstechnische Brenntemperatur der Keramik über den Anteil an B₂O₃, Li₂O und Na₂O gesteuert und gewünschten Werten angepasst werden. Die anwendungstechnisch relevante Brenntemperatur der erfindungsgemäßen Keramiken liegt im Bereich von 870 bis 970 °C.

Insgesamt wird eine Glaskeramik zur Verfügung gestellt, die bezüglich ästhetischer Verblendkeramik sämtlichen Anforderungen genügt.

Ein Verfahren zur Herstellung der erfindungsgemäßen Glaskeramik zeichnet sich durch folgende Verfahrensschritte aus:
1.) Einwaage der Komponenten gemäß einem der Ansprüche 1 bis 5;
2.) Mischen des Gemenges vorzugsweise in einem Rhönradmischer;
3.) Schmelzen des Gemenges in einem vorzugsweise gasbeheizten Ofen wie Tropftiegelofen bei vorzugsweise ca. 1500 °C;
4.) Abschrecken der aus dem Ofen heraustretenden Schmelze in einem Wasserbad und anschließendes Trocknen;
5.) kurzzeitiges Mahlen der so erhaltenen Fritte in einer Mühle (z. B. Kugelmühle);
6.) Tempern der Fritte;
7.) Einfüllen der Fritte nach Trocknung in eine Mühle wie Kugelmühle und Mahlen bis vorzugsweise ca. 10000 Umdrehungen;
8.) Sieben der gemahlenen Fritte durch ein Sieb, wobei der Siebdurchgang das Endprodukt bildet.

Vorzugsweise wird das Tempern der Fritte auf folgende Weise durchgeführt:
a. Aufschichten der gemahlenen Fritte auf quarzbeschichtete Schamottplatten.
b. Einstellen der Schamottplatten in einen auf ca. 960 °C aufgeheizten Ofen (z. B. Elektroofen),
c. Entnahme der Platten aus dem Ofen nach ca. 40 Minuten,
d. Abschrecken der zusammengeschmolzenen Frittekuchen in einem Wasserbad.

Die gemahlene Fritte wird bevorzugt durch ein Sieb mit einer Maschenweite M im Bereich von 80 µm ≤ M ≤ 120 µm vorzugsweise M = 100 µm gesiebt.

Im Unterschied zu der aus EP 0 622 342 B1 bekannten Glaskeramik kommt die erfindungsgemäße Glaskeramik ohne ZrO₂ und TiO₂ aus und der Me (II)O-Gehalt bleibt unterhalb von 3 Gew.-%. Durch Einschmelzung von CeO₂ und Tb₂O₃ hat die erfindungsgemäße Opalkeramik außerdem Fluoreszenz. Ferner kann die Brenntemperatur an den gewünschten Anwendungszweck angepasst werden. Durch gezielte Leucit-Kristallisation lässt sich bei der erfindungsgemäßen Keramik die Wärmedehnung so einstellen, das sie insbesondere zur Verblendung metallischer Gerüstwerkstoffe einsetzbar ist.

Weitere Einzelheiten, Vorteile und Merkmale der Erfindung ergeben sich nicht nur aus den Ansprüchen, den diesen zu entnehmenden Merkmalen -für sich und/oder in Kombination-, sondern auch aus den der nachfolgenden Beschreibung zu entnehmenden bevorzugten Ausführungsbeispielen.

Die Erfindung wird anhand nachstehender Ausführungsbeispiele erläutert, wobei die Proben 1, 2, 8 bis 21 als bevorzugt und die Proben 11 und 13 als besonders bevorzugt zu bezeichnen sind. Die Zusammensetzungen der Opalkeramik sind der Tabelle I zu entnehmen.

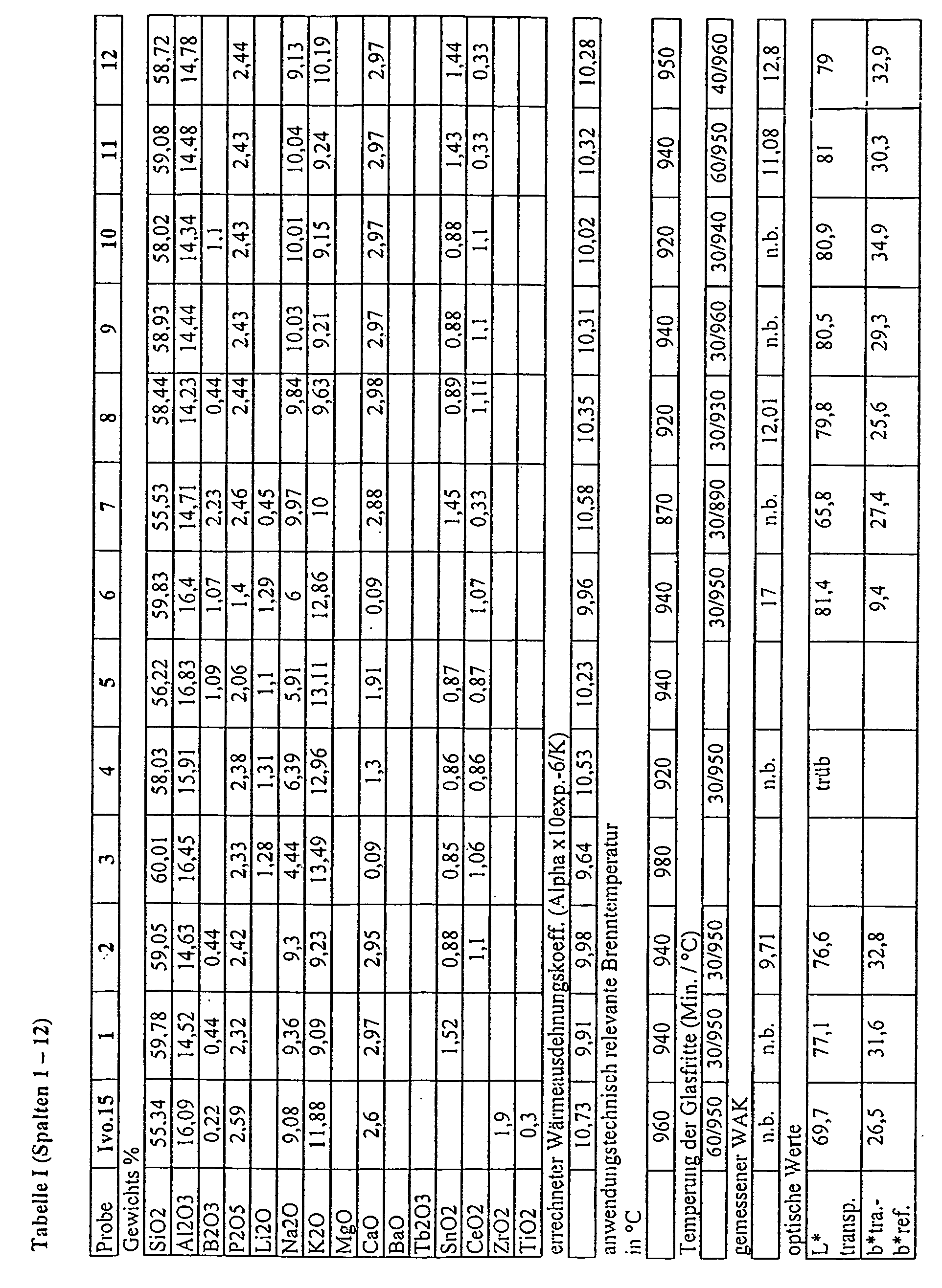

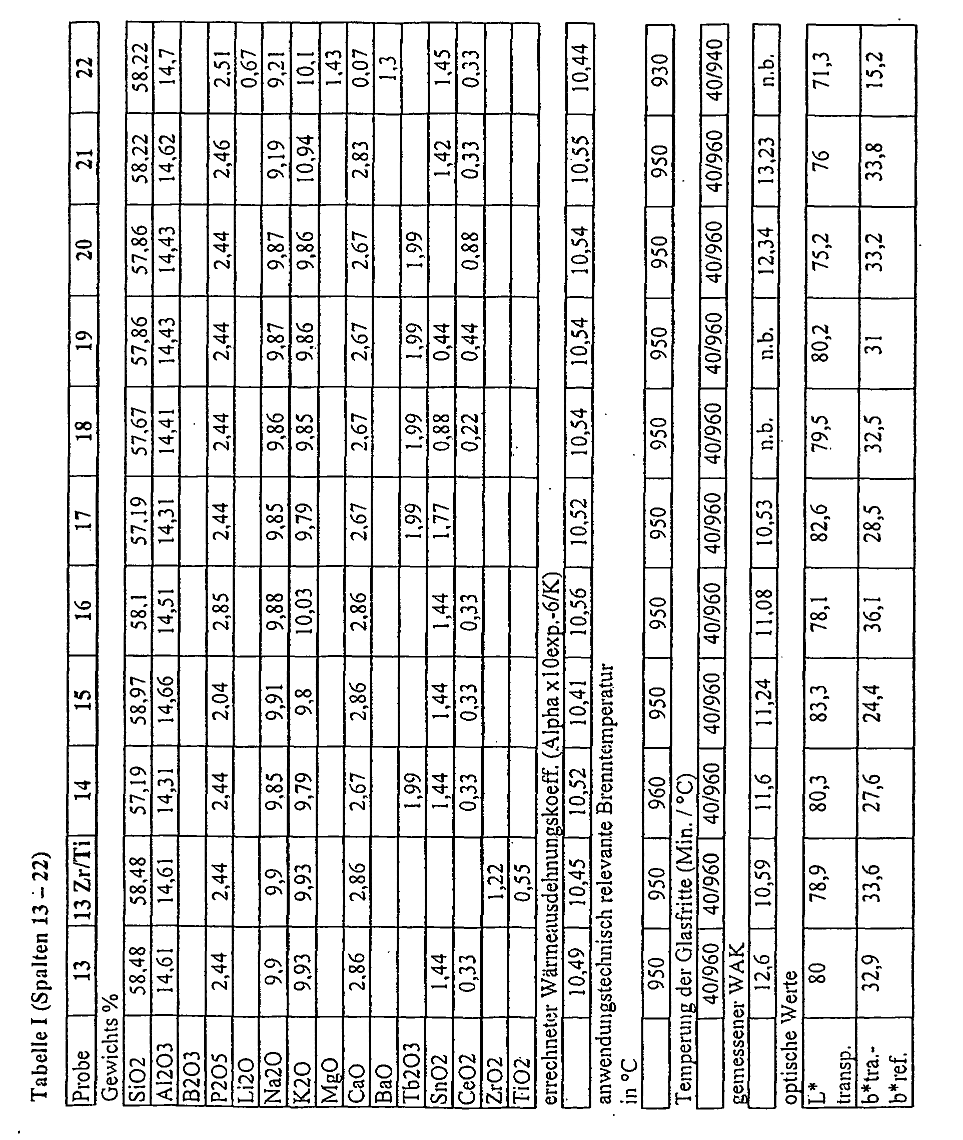

Ein Vergleich der Proben zeigt, dass der Wärmeausdehnungskoeffizient der erfindungsgemäßen Glaskeramik steuerbar ist. So kann ein Wärmeausdehnungskoeffizient (WAK) im Bereich von 9,0 bis 13, 5 x 10 ⁻⁶/K eingestellt werden. Im Vergleich zu den aus EP 0 622 342 B1 bekannten Keramiken, die entweder einen zu geringen WAK (≤ 11 x 10⁻⁶/K) oder einen zu hohen WAK (≥ 16 x 10⁻⁶/K) aufweisen, kann die erfindungsgemäße Glaskeramik in den im Dentalbereich besonders wichtigen WAK-Bereich von 11,0 bis 13,0 x 10⁻⁶/K für Verblendkeramiken eingestellt werden.

Versuche haben gezeigt, dass der SnO₂ - und/oder CeO₂ - Gehalt geeignet ist, die Kristallisation geringer Mengen an Leucit anzuregen, die für die Anhebung des WAK notwendig ist.

Ein Vergleich der Probe 13 mit 1,44 % SnO₂ mit einer Probe, in welcher entsprechend den Ansprüchen aus EP 0 622 342 B1 das SnO₂ durch ZrO₂ und TiO₂ ausgetauscht wurde (Probe "13 Zr/Ti" in Tabelle), zeigt deutlich, dass bei letzterer Probe der WAK bei Annahme eines homogenen Glases ungefähr dem rechnerisch ermittelten Wert entspricht, was als Fehlen einer Leucit-Kristallisation gedeutet werden kann.

Die Beispiele zeigen, dass der WAK bei der erfindungsgemäßen Glaskeramik bei ausreichendem Al₂O₃ - Gehalt über den K₂O - Gehalt gesteuert werden kann. Diese Eigenschaft zeigen insbesondere die Proben 11 bis 14 und 21. Die Proben 15 und 16 verdeutlichen, dass auch der P₂O₅ - Gehalt, der das Maß der Phasentrennung des Glases bestimmt, Einfluss hat.

Durch zusätzliches Einschmelzen von CeO₂ und/oder Tb₂O₃ kann erreicht werden, dass die Glaskeramik fluoresziert. Dabei konnte festgestellt werden, dass die Einschmelzung von lediglich CeO₂ zu schwach bläulicher Fluoreszenz und von Tb₂O₂ zu einer stärkeren, aber für natürliche Zähne untypisch gelben Fluoreszenz führt. Nur mit einer Kombination beider Oxide wird eine starke, neutrale Fluoreszenz erreicht, wie dies die Proben 17 bis 20 zeigen. Die Proben 19 und 20 sind für die Fluoreszenz optimal.

Die Brenntemperatur der Glaskeramik kann über den Anteil an B₂O₃, Li₂O und Na₂O gesteuert und dem gewünschten Wert angepasst werden, wie dies die Proben 10 und 12 zeigen. Die anwendungstechnisch relevanten Brenntemperaturen der erfindungsgemäßen Glaskeramiken liegen im Bereich von 870 °C bis 970 °C.

Die Probe 13 hat sämtliche bevorzugten Eigenschaften. Die Brenntemperatur und der WAK sind ideal und gleichzeitig sind die optischen Werte mit einer Transparenz von L* (Transparenz) = 80 und einem Δb* = 32,9 sehr gut. Im Vergleich dazu hat die gemäß EP 0 622 342 B1 nachvollzogene Keramik (Ivo. 15), nur ein Δb* von 26,5 bei einer Transparenz von L* (transp.) = 69,7.

Es ist zu erwähnen, dass die chemische Beständigkeit und Biegefestigkeit der dargestellten Beispiele die Forderungen der maßgeblichen Normen für Dentalkeramiken (ISO 9693) erfüllen.

Die beispielhaften Ausführungen gemäß der Proben 13, 15 und 16 zeigen den Einfluss des P₂O₅ - Gehaltes. Beispiel 13 mit einem P₂O₅-Gehalt von 2,44 Gew.-% wird als optimal angesehen, während bei dem Beispiel 15 mit einem P₂O₅-Gehalt von 2,04 Gew.-% die Transparenz auf Kosten der Opaleszenz erhöht ist und bei Beispiel 16 mit einem P₂O₅-Gehalt von 2,85 Gew.-% eine erhöhte Opaleszenz bei verminderter Transparenz festzustellen ist.

Die Tabelle I zeigt auch Ausführungen (Proben 3, 4, 5, 6, 7 und 22), deren Zusammensetzungen außerhalb des beanspruchten Bereichs liegen. Dabei haben die Proben 3 bis 5 einen zu hohen K₂O―Gehalt und Probe 3 zusätzlich zu wenig CaO. Es hat sich gezeigt, dass diese beispielhaften Ausführungen schon im ungetemperten Zustand - vermutlich wegen zu starker Leucitkristallisation - zu trübe sind. Probe 6 ist trotz hohem Leucitgehaltes zwar transparent, da es wegen eines P₂O₅-Gehalls von unter 1,5 Gew.-% nur geringe Neigung zur Entmischung hat, ist ihre Opaleszenz aber zu gering. Bei Probe 7 führt ein zu hoher B₂O₃-Gehalt zu einer zu starken Trübung und bei Probe 22 wurde CaO durch MgO und BaO ersetzt, was zu einer verminderten Opaleszenz führt.

Die erfindungsgemäßen Glaskeramiken wurden gemäß folgendem Herstellungsverfahren hergestellt:
1. Einwaage der Rohstoffe gemäße Angabe in Tabelle 1.
2. Mischen des Gemenges in einem Rhönradmischer.
3. Schmelzen des Gemenges in einem gasbeheizten Tropftiegelofen bei ca. 1500 °C.
4. Abschrecken der aus dem Ofen herauslaufenden Schmelze in einem Wasserbad.
5. Trocknen der Schmelze.
6. Kurzzeitiges Mahlen der so erhaltenen Fritte in einer Kugelmühle.
7. Tempern der Fritte auf folgende Weise:
   - Aufschichten der gemahlenen Fritte auf quarzbeschichtete Schamottplatten
   - Einstellen dieser Platten in einen auf 960 °C aufgeheizten Elektroofen
   - Entnahme der Platten nach ca. 40 Minuten aus dem Ofen
   - Abschrecken des zusammengeschmolzenen Frittekuchen im Wasserbad.
8. Einfüllen der Fritte nach Trocknung in die Kugelmühle und Mahlen bis vorzugsweise ca. 10000 Umdrehungen.
9. Sieben der gemahlenen Fritte durch ein Sieb mit vorzugsweise 100 µm Maschenweite.

Zur Messung von Opaleszenz und Transparenz der Opalkeramik ist anzumerken, dass 3 Gramm der pulverförmigen Opalkeramik in einer Presse zu einer Ronde verdichtet wurden und diese bei der Anwendungstemperatur wie bei der Probe 13 von 950 °C in einem zahntechnischen Vakuumbrennofen zusammengesintert wurde. Diese so erhaltene Ronde hat eine Dicke von ca. 2,5 mm. Im Falle der Opaleszenz erscheinen diese Ronden bei Durchsicht orange-gelb, da vorrangig das nicht gestreute langwellige Licht ins Auge fällt. Bei Aufsicht gegen einen dunklen Hintergrund hingegen erscheint die Ronde bläulich, da in diesem Fall das stärker gestreute kurzwellige Licht gesehen wird.

Dieses Phänomen lässt sich mit einem Spektralphotometer quantitativ bestimmen. Hierzu werden im Photometer beispielsweise der Firma Minolta (CM-3610d) die L*,a*,b*-Werte in Transmission und in Reflexion auf schwarzem Grund gemessen, wobei ein 2°-Beobachter und Normlicht D65 vorausgesetzt werden. Im L*,a*,b*-Farbsystem steht ein positiver b*-Wert für den Gelbanteil des Lichtes, ein negativer b*-Wert hingegen für seinen Blauanteil. Je höher der absolute Betrag, desto intensiver die Farbe.

Die opaleszierenden Ronden ergeben folglich bei Transmission positive- und bei Reflexion negative b*-Werte. Je mehr die beiden b*-Werte voneinander abweichen, desto stärker ist die Opaleszenz.

Der in Transmission gemessene L*-Wert kann darüberhinaus als Maß für die Transparenz der Ronde verwendet werden.

Ästhetisch befriedigende Resultate bei Zahnrestaurationen werden mit Opalkeramiken erreicht, deren Ronden ein Δb* von mindestens 25 und L* (transm.) von mindestens 75 aufweisen.

## Patentansprüche

1. Opaleszierende Glaskeramik, insbesondere eine opaleszierende Glaskeramik als Dentalmaterial oder als Zusatz zu oder Bestandteil von Dentalmaterial, enthaltend zumindest die Komponenten SiO₂, Al₂O₃, P₂O₅, Na₂O, K₂O, CaO sowie Me(IV)O₂,
**dadurch gekennzeichnet,**
**dass** die opaleszierende Keramik ZrO₂ und TiO₂ frei ist, dass die Glaskeramik einen Me(II)O-Gehalt von weniger als 4 Gew.-% aufweist und dass der Me(IV)O₂ - Gehalt 0,5 bis 3 Gew.-% beträgt.

2. Opaleszierende Glaskeramik nach Anspruch 1,
**dadurch gekennzeichnet,**
**dass** sich Me(IV)O₂ aus 0 - 1 Gew.-% CeO₂ und 0 - 2,5 Gew.-% SnO₂ zusammensetzt.

3. Opaleszierende Glaskeramik nach Anspruch 1,
**dadurch gekennzeichnet,**
**dass** der Me (II)O-Gehalt 2 - 3,5 Gew.-%, insbesondere 2,5 - 3 Gew.-% beträgt.

4. Opaleszierende Glaskeramik nach einem der Ansprüche 1 bis 3,
**dadurch gekennzeichnet,**
**dass** die Glaskeramik folgende Komponenten enthält:
| **Komponente** | **Gew.-%** |
|---|---|
| SiO₂ | 55 - 62 |
| Al₂O₃ | 13 - 17 |
| B₂O₃ | 0-2 |
| P₂O₅ | 1,5 - 3 |
| Li₂O | 0 - 2 |
| Na₂O | 7-12 |
| K₂O | 8-12 |
| MgO | 0 - 2 |
| CaO | 1 - 4 |
| BaO | 0 - 2 |
| Tb₂O₃ | 0 - 3 |
| Me(IV)O₂ | 0,5 - 3 |
wobei die angegebene Menge Me(IV)O₂ aus 0 - 1 Gew.-% CeO₂ und 0 - 2,5 Gew.-% SnO₂ gebildet ist.

5. Opaleszierende Glaskeramik nach einem der Ansprüche 1 bis 3,
**dadurch gekennzeichnet,**
**dass** die Glaskeramik folgende Komponenten enthält:
| **Komponente** | **Gew.-%** |
|---|---|
| SiO₂ | 58 - 60 |
| Al₂O₃ | 14 - 15 |
| P₂O₅ | 2,3 - 2,6 |
| Na₂O | 9,5 -10,5 |
| K₂O | 9 - 10 |
| CaO | 2,8 - 3,0 |
| SnO₂ | 1,3 - 1,6 |
| CeO₂ | 0,3 - 0,4 |
| Tb₂O₃ | 0-2,0 |

6. Opaleszierende Glaskeramik nach zumindest einem der vorhergehenden Ansprüche,
**dadurch gekennzeichnet,**
**dass** zur Erzielung einer fluoreszierender Eigenschaft CeO₂ und/oder Tb₂O₃ eingeschmolzen ist.

7. Opaleszierende Glaskeramik nach zumindest einem der vorhergehenden Ansprüche,
**dadurch gekennzeichnet,**
**dass** die Glaskeramik einen Wärmeausdehnungskoeffizienten (WAK) im Bereich von 9,0 - 13,5 x 10⁻⁶/K, insbesondere 10,5- 12,0 x 10⁻⁶/K aufweist.

8. Verfahren zur Herstellung einer opaleszierenden Gläskeramik nach einem der Ansprüche 1 bis 5,
**dadurch gekennzeichnet,**
**dass** das Verfahren folgende Verfahrensschritte umfasst:
- Einwaage und Mischen der Komponenten mit einem Mischungsverhältnis gemäß einem der Ansprüche 1 bis 5;
- Schmelzen des Gemenges in einem Ofen;
- Abschrecken der aus dem Ofen heraustretenden Schmelze in einem Wasserbad und anschließendes Trocknen;
- Mahlen der so erhaltenen Fritte in einer Mühle;
- Tempern der Fritte;
- Abfüllen der Fritte nach Trocknung in eine Mühle und Mahlen der Fritte;
- Sieben der gemahlenen Fritte durch ein Sieb, wobei der Siebdurchgang das Endprodukt bildet.

9. Verfahren nach Anspruch 8,
**dadurch gekennzeichnet,**
**dass** das Tempern der Fritte in folgender Weise durchgeführt wird:
- Aufschichten der gemahlenen Fritte auf quarzbeschichtete Schamottplatten,
- Einstellen der Schamottplatten in einen auf eine Temperatur T mit 850 °C ≤ T ≤ 1000°C aufgeheizten Ofen wie Elektroofen,
- Entnahme der Platten aus dem Ofen nach einer Zeit t mit 30 min ≤ t ≤ 60,
- Abschrecken der zusammengeschmolzenen Frittekuchen in einem Wasserbad.

10. Verfahren nach Anspruch 8 oder 9,
**dadurch gekennzeichnet,**
**dass** die Komponenten in einem Rhönradmischer gemischt werden

11. Verfahren nach zumindest einem der Ansprüche 8 bis 10,
**dadurch gekennzeichnet,**
**dass** das Gemenge in einem vorzugsweise gasbeheizten Tropftiegelofen geschmolzen wird.

12. Verfahren nach zumindest einem der Ansprüche 8 bis 11,
**dadurch gekennzeichnet,**
**dass** die Fritte nach Trocknung in eine Kugelmühle abgefüllt und mit etwa 10 000 Umdrehungen pro Minute gemahlen wird.

13. Verfahren nach zumindest einem der Ansprüche 8 bis 12,
**dadurch gekennzeichnet ,**
**dass** die gemahlene Fritte vorzugsweise durch ein Sieb mit einer Maschenweite M im Bereich von 80 µm ≤ M ≤ 120 µm vorzugsweise M = 100 µm gesiebt wird.

14. Verfahren nach zumindest einem der Ansprüche 8 bis 13,
**dadurch gekennzeichnet,**
**dass** das Zusammensintern durch Erwärmen des Granulats auf 870 bis 970 °C bewirkt wird.

15. Verfahren nach zumindest einem der Ansprüche 8 bis 14,
**dadurch gekennzeichnet,**
**dass** der Wärmeausdehnungskoeffizient (WAK) durch den K₂O-Gehalt auf einen Wert 9,0 ≤ WAK ≤ 13,5 x 10⁻⁶/K eingestellt wird.

16. Verfahren nach zumindest einem der Ansprüche 8 bis 15,
**dadurch gekennzeichnet,**
**dass** die Brenntemperatur der opaleszierenden Glaskeramik über Anteile von B₂O₃, Li₂O und Na₂O gesteuert wird und vorzugsweise im Bereich von 870 °C bis 970 °C liegt.

17. Verwendung der opaleszierenden Glaskeramik nach zumindest einem der vorhergehenden Ansprüche als Dentalmaterial oder als Zusatz zu oder Bestandteil von Dentalmaterial.

18. Verwendung nach Anspruch 17, wobei das opaleszierende Glas Bestandteil von Inlays, Onlays, Brücken oder Kronen ist.

## Claims

1. An opalescent glass ceramic, in particular an opalescent glass ceramic as a dental material or as an additive to or component of dental material, comprising at least the components SiO₂, Al₂O₃, P₂O₅, Na₂O, K₂O, CaO and Me(IV)O₂,
**characterized in**
**that** the opalescent ceramic is devoid of ZrO₂ and TiO₂, that the glass ceramic has a Me(II)O content of less than 4% by weight and that the Me(IV)O₂ content is 0.5 to 3% by weight.

2. The opalescent glass ceramic according to claim 1,
**characterized in**
**that** Me(IV)O₂ is composed of 0 - 1% by weight CeO₂ and 0 - 2.5% by weight SnO₂.

3. The opalescent glass ceramic according to claim 1,
**characterized in**
**that** the Me(II)O content is 2 - 3.5% by weight, in particular 2.5 -3% by weight.

4. The opalescent glass ceramic according to any one of the claims 1 to 3,
**characterized in**
**that** the glass ceramic contains the following components:
| **Component** | **% by weight** |
|---|---|
| SiO₂ | 55 - 62 |
| Al₂O₃ | 13 - 17 |
| B₂O₃ | 0 - 2 |
| P₂O₅ | 1.5 - 3 |
| Li₂O | 0 - 2 |
| Na₂O | 7 - 12 |
| K₂O | 8 - 12 |
| MgO | 0-2 |
| CaO | 1-4 |
| BaO | 0-2 |
| Tb₂O₃ | 0 - 3 |
| Me(IV)O₂ | 0.5 - 3 |
the indicated amount of Me(IV)O₂ being composed of 0 - 1% by weight CeO₂ and 0 - 2.5% by weight SnO₂.

5. The opalescent glass ceramic according to any one of the claims 1 to 3,
**characterized in**
**that** the glass ceramic contains the following components:
| **Component** | **% by weight** |
|---|---|
| SiO₂ | 58 - 60 |
| Al₂O₃ | 14 - 15 |
| P₂O₅ | 2.3 - 2.6 |
| Na₂O | 9.5 - 10.5 |
| K₂O | 9 - 10 |
| CaO | 2.8 - 3.0 |
| SnO₂ | 1.3 - 1.6 |
| CeO₂ | 0.3 - 0.4 |
| Tb₂O₃ | 0 - 2.0 |

6. The opalescent glass ceramic according to at least one of the preceding claims,
**characterized in**
**that** CeO₂ and/or Tb₂O₃ are fused to obtain a fluorescent property.

7. The opalescent glass ceramic according to at least one of the preceding claims,
**characterized in**
**that** the glass ceramic has a thermal expansion coefficient (TEC) in the range of 9.0 - 13.5 x 10⁻⁶/K, in particular 10.5 -12.0 x 10⁻⁶/K.

8. A method for producing an opalescent glass ceramic according to any one of the claims 1 to 5,
**characterized in**
**that** the method comprises the following procedural steps:
- weighing in and mixing the components with a mixing ratio according to one of the claims 1 to 5;
- melting the mixture in a furnace;
- quenching the molten mass coming out of the furnace in a water bath and subsequent drying;
- grinding the frit thus obtained in a mill;
- tempering the frit;
- after drying, filling the frit in a mill and grinding the frit;
- sifting the ground frit through a sieve, the sieve opening forming the end.

9. The method according to claim 8,
**characterized in**
**that** the tempering of the frit is carried out in the following manner:
- stacking the ground frits on quartz-coated fireclay plates,
- placing the fireproof plates in a furnace, e.g. an electric furnace, heated to a temperature T with 850°C ≤ T ≤ 1000°C,
- removing the plates from the furnace after a time t with 30 min ≤ t ≤ 60,
- quenching the melted frit cakes in a water bath.

10. The method according to claim 8 or 9,
**characterized in**
**that** the components are mixed in a drum hoop mixer (Rhönradmischer).

11. The method according to at least one of the claims 8 to 10,
**characterized in**
**that** the mixture is melted in a preferably gas-heated drip-feed crucible furnace.

12. The method according to at least one of the claims 8 to 11,
**characterized in**
**that** after drying, the frit is filled into a ball mill and ground with about 10,000 revolutions per minute.

13. The method according to at least one of the claims 8 to 12,
**characterized in**
**that** the ground frit is preferably sifted through a sieve having a mesh size M in the range of 80 µm ≤ M ≤ 120 µm, preferably M = 100 µm.

14. The method according to at least one of the claims 8 to 13,
**characterized in**
**that** the sintering is produced by heating the granulated material to 870 to 970°C.

15. The method according to at least one of the claims 8 to 14,
**characterized in**
**that** the thermal expansion coefficient (TEC) is set to a value 9.0 ≤ TEC ≤ 13.5 x 10⁻⁶/K by the K₂O content.

16. The method according to at least one of the claims 8 to 15,
**characterized in**
**that** the firing temperature of the opalescent glass ceramic is controlled by the proportions of B₂O₃, Li₂O and Na₂O and is preferably in the range of 870°C to 970°C.

17. Use of the opalescent glass ceramic according to at least one of the preceding claims as a dental material or as an additive to or component of dental material.

18. The use according to claim 17, wherein the opalescent glass is a component of inlays, onlays, bridges or crowns.

## Revendications

1. Vitrocéramique opalescente, en particulier vitrocéramique opalescente en tant que matériel dentaire ou additif pour matériel dentaire ou constituant de matériel dentaire, contenant au moins les composants SiO₂, Al₂O₃, P₂O₅, Na₂O, K₂O, CaO ainsi que Me(IV)O₂,
**caractérisée en ce que**
la céramique opalescente est exempte de Zr02 et TiO₂, la vitrocéramique présente une teneur en Me(II)O inférieure à 4 % en poids et la teneur en Me(IV)O₂ est de 0,5 à 3 % en poids.

2. Vitrocéramique opalescente selon la revendication 1,
**caractérisée en ce que**
le Me(IV)O₂ est constitué de 0 à 1 % en poids de CeO₂ et de 0 à 2,5 % en poids de SnO₂.

3. Vitrocéramique opalescente selon la revendication 1,
**caractérisée en ce que**
la teneur en Me(II)O est de 2 à 3,5 % en poids, en particulier de 2,5 à 3 % en poids.

4. Vitrocéramique opalescente selon l'une quelconque des revendications 1 à 3,
**caractérisée en ce que**
la vitrocéramique contient les composants suivants :
| Composants | % en poids |
|---|---|
| SiO₂ | 55 - 62 |
| Al2₃O₃ | 13 - 17 |
| B₂O₃ | 0 - 2 |
| P₂O₅ | 1,5 - 3 |
| Li₂O | 0 - 2 |
| Na₂O | 7 - 12 |
| K₂O | 8 - 12 |
| MgO | 0-2 |
| CaO | 1 - 4 |
| BaO | 0 - 2 |
| Tb₂O₃ | 0 - 3 |
| Me(IV)O₂ | 0,5 - 3 |
dans laquelle la quantité de Me(IV)O₂ indiquée est constituée de 0 à 1 % en poids de CeO₂ et de 0 à 2,5 % en poids de SnO₂.

5. Vitrocéramique opalescente selon l'une quelconque des revendications 1 à 3,
**caractérisée en ce que**
la vitrocéramique contient les composants suivants :
| Composants | % en poids |
|---|---|
| SiO₂ | 58 - 60 |
| Al2₃O₃ | 14 - 15 |
| P₂O₅ | 2,3 - 2,6 |
| Na₂O | 9,5 - 10,5 |
| K₂O | 9 - 10 |
| CaO | 2,8 - 3,0 |
| SnO₂ | 1,3 - 1,6 |
| CeO₂ | 0,3 - 0,4 |
| Tb₂O₃ | 0 - 2,0 |

6. Vitrocéramique opalescente selon au moins l'une quelconque des revendications précédentes,
**caractérisée en ce qu'**
on fait fondre du Ce02 et/ou Tb₂O₃ pour obtenir une propriété fluorescente.

7. Vitrocéramique opalescente selon au moins l'une quelconque des revendications précédentes,
**caractérisée en ce qu'**
elle présente un coefficient de dilation thermique (CDT) de l'ordre de 9,0 à 13,5 x 10⁶/K, en particulier de 10,5 à 12,0 x 10⁻⁶/K.

8. Procédé de fabrication d'une vitrocéramique opalescente selon l'une quelconque des revendications 1 à 5,
**caractérisé en ce qu'**
il comporte les étapes suivantes :
- pesée et mélange des composants dans un rapport de mélange selon l'une quelconque des revendications 1 à 5 ;
- fusion du mélange dans un four ;
- trempe de la masse fondue sortant du four dans un bain d'eau puis séchage ;
- broyage de la fritte ainsi obtenue dans un broyeur ;
- recuit de la fritte ;
- transfert de la fritte, après séchage, dans un broyeur et broyage de la fritte ;
- tamisage de la fritte broyée dans un tamis, le tamisat constituant le produit final.

9. Procédé selon la revendication 8,
**caractérisé en ce que**
le recuit de la fritte est réalisé de la façon suivante :
- empilage de la fritte broyée sur des plaques réfractaires recouvertes de quartz,
- mise en place des plaques réfractaires dans un four chauffé à une température T de 850°C ≤ T ≤ 1000°C, comme un four électrique,
- prélèvement des plaques du four au bout d'un temps t de 30 min ≤ t ≤ 60,
- trempe du gâteau de frittage fondu, dans un bain d'eau.

10. Procédé selon la revendication 8 ou 9,
**caractérisé en ce que**
les composants sont mélangés dans un mélangeur Röhnrad.

11. Procédé selon au moins l'une quelconque des revendications 8 à 10,
**caractérisé en ce que**
le mélange est fondu dans un four à creusets de préférence chauffé au gaz.

12. Procédé selon au moins l'une quelconque des revendications 8 à 11,
**caractérisé en ce que**
la fritte est versée, après séchage, dans un broyeur à galets et broyée à environ 10 000 tours par minute.

13. Procédé selon au moins l'une quelconque des revendications 8 à 12,
**caractérisé en ce que**
la fritte broyée est tamisée de préférence dans un tamis d'une largeur de maille M de l'ordre de 80 µm ≤ M ≤ 120 µm, de préférence M = 100 µm.

14. Procédé selon au moins l'une quelconque des revendications 8 à 13,
**caractérisé en ce que**
le frittage est provoqué par chauffage du granulat à une température de 870 à 970°C.

15. Procédé selon au moins l'une quelconque des revendications 8 à 14,
**caractérisé en ce que**
le coefficient de dilatation thermique (CDT) est ajusté, grâce à la teneur en K₂O, à une valeur de 9,0 ≤ CDT ≤ 13,5 x 10⁻⁶/K.

16. Procédé selon au moins l'une quelconque des revendications 8 à 15,
**caractérisé en ce que**
la température de combustion de la vitrocéramique opalescente est pilotée à l'aide de fractions de B₂O₃, de Li₂O et de Na₂O et, de préférence, à une température de l'ordre de 870°C à 970°C.

17. Utilisation de la vitrocéramique opalescente selon au moins l'une quelconque des revendications précédentes, en tant que matériel dentaire ou en tant qu'additif pour matériel dentaire ou constituant d'un matériel dentaire.

18. Utilisation selon la revendication 17, le verre opalescent étant un constituant de pansement interne, (inlay), de pansement externe (on-lay), de bridge ou de couronne.
